(19) Europäisches Patentamt
European Patent Office
Office européen des brevets

(11) Numéro de publication: **0 117 779**
**B1**

# (12) FASCICULE DE BREVET EUROPÉEN

(45) Date de publication du fascicule du brevet: **06.12.89**

(21) Numéro de dépôt: **84400156.0**

(22) Date de dépôt: **25.01.84**

(51) Int. Cl.⁴: **C 07 D 237/20,** A 61 K 31/50, C 07 D 237/24, C 07 D 403/04, C 07 D 409/04

ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | (SEITE) | (SPALTE) | (ZEILE) | LAUTET BERICHTIGT:<br>SHOULD READ :<br>DEVRAIT ETRE LU : |
|---|---|---|---|---|
| 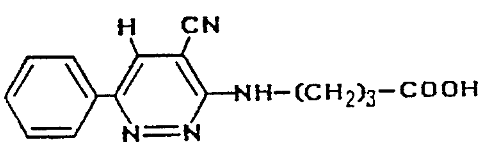 | 5 | | 61/65 | 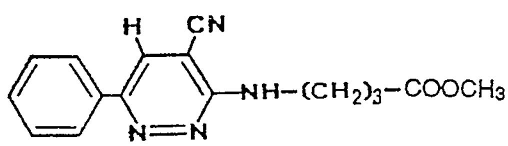 |

Tag der Entscheidung über die Berichtigung / Date of decision on rectification: / Date de décision portant sur modification: 4.4.90

Ausgabe- und Veröffentlichungstag: / Issue and publication date: / Date d'edition et de publication: 13.6.90

Patbl.Nr / EPB no: 90/24 / Bull. no:

(19) Europäisches Patentamt
European Patent Office
Office européen des brevets

(11) Numéro de publication: **0 117 779**
**B1**

# (12) FASCICULE DE BREVET EUROPÉEN

(45) Date de publication du fascicule du brevet: **06.12.89**

(21) Numéro de dépôt: **84400156.0**

(22) Date de dépôt: **25.01.84**

(51) Int. Cl.⁴: **C 07 D 237/20,** A 61 K 31/50, C 07 D 237/24, C 07 D 403/04, C 07 D 409/04

(54) **Dérivés de la pyridazine actifs sur le système nerveux central, procédé d'obtention et médicaments en contenant.**

(30) Priorité: **27.01.83 FR 8301234**

(43) Date de publication de la demande: **05.09.84 Bulletin 84/36**

(45) Mention de la délivrance du brevet: **06.12.89 Bulletin 89/49**

(84) Etats contractants désignés: **AT BE CH DE FR GB IT LI LU NL SE**

(56) Documents cité:
**EUR. J. MED. CHEM. - CHIMICA THERAPEUTICA, vol. 11, no. 2, mars-avril 1976, pages 107-113, Paris, FR. G. LECLERC et al.: "Syntheses et correlations de Hansch dans une serie d'hydrazionopyridazines hypotensives"**
**Brevet francais FR CEPBE no. A 2141697**

**Le dossier contient des informations techniques présentées postérieurement au dépôt de la demande et ne figurant pas dans le présent fascicule.**

(73) Titulaire: **SANOFI S.A., 40, Avenue George V, F-75008 Paris (FR)**

(72) Inventeur: **Chambon, Jean- Pierre, Chemin des Truquets Route de Montpellier, F-34570 Montarnaud (FR)**
Inventeur: **Biziere, Kathleen, 6, Lotissements Rayons d'Oc, F-34170 Clapiers (FR)**
Inventeur: **Wermuth, Camille- Georges, 3, rue de la Côte d'Azur, F-67100 Strasbourg (FR)**

(74) Mandataire: **Gillard, Marie- Louise, Cabinet Beau de Loménie 55, Rue d'Amsterdam, F-75008 Paris (FR)**

EP 0 117 779 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne, en tant que produits nouveaux, des dérivés de la pyridazine.

Elle concerne également un procédé de préparation de ces composés et les médicaments qui contiennent, comme principe actif, au moins un desdits dérivés.

Les composés selon l'invention répondent à la formule générale:

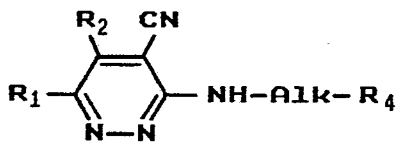

(I)

dans laquelle

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$ ou un groupe phényle;

Alk représente un groupe $(CH_2)_n$ où *n* est un entier égal à 2, 3, 4 ou encore un groupe 1,2–propynyle–$CH_2$–C≡C–

$R_4$ représente: –COOH
–COO–alkyle en $C_1$–$C_4$
–$CONH_2$
–C≡N

Les composés (I) sont susceptibles de donner avec les acides minéraux ou organiques des sels d'addition. La présente invention concerne également les sels d'addition que fournissent les composés (I) avec les acides pharmaceutiquement acceptables.

Les composés selon l'invention peuvent être obtenus à partir des chloro–3 pyridazines convenablement substitués *1* selon le schéma réactionnel suivant:

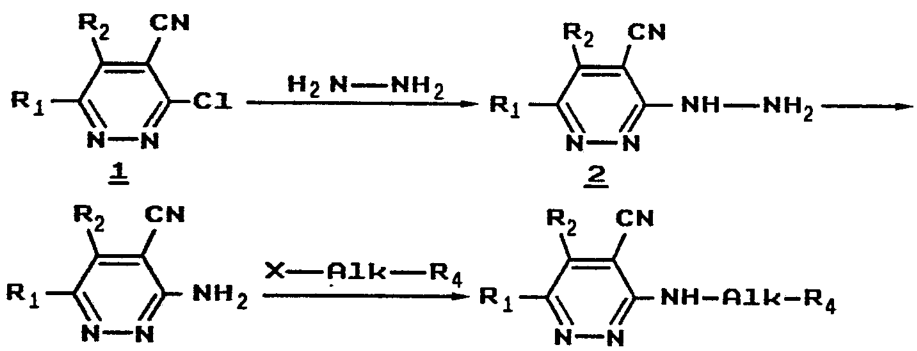


3

I ($R_4$ = COO–alkyle en $C_1$–$C_4$ ou CN)

A partir des pyridazines chlorées en position 3, on prépare les amino–3 pyridazines correspondantes *3*. Dans la pratique, le passage direct des dérivés chlorés aux dérivés aminés s'avérant impraticable, on passe par l'intermédiaire des dérivés hydrazinés *2* obtenus avec de bons rendements par chauffage au reflux des dérivés chlorés *1* avec un excès d'hydrate d'hydrazine. Les composés *2* par hydrogénation en présence d'un catalyseur, tel que le nickel de Raney, conduisent aux composés *3*.

Par action sur *3* d'un ester ω–halogéné X–Alk–$R_4$ où X représente un halogène, de préférence le brome, et $R_4$ représente un groupe COO–alkyle en $C_1$–$C_4$ ou le groupe cyano, on obtient les composés (I) où $R_4$ représente un groupe COO–alkyle en $C_1$–$C_4$ ou un groupe cyano. On effectue la réaction par chauffage des réactifs au sein d'un solvant, tel que le diméthylformamide, à une température comprise entre 50 et 100°C.

Les composés (I) où $R_4$ représente un groupe –COOH sont obtenus à partir des composés où $R_4$ représente un groupe –COO-alkyle en $C_1$–$C_4$ par saponification en milieu acide, de préférence par chauffage avec un hydracide, tel que l'acide chlorhydrique ou l'acide bromhydrique, au sein de l'acide acétique à une température comprise entre 20 et 100°C. On isole l'acide directement sous forme du sel correspondant à l'hydracide utilisé, par évaporation à siccité.

Enfin, les composés (I) où $R_4$ représente un groupe carboxamido peuvent être préparés, soit à partir des esters (I) correspondants par action de l'ammoniac en solution dans un alcool aliphatique, tel que le méthanol, soit à partir des nitriles (I).

Dans le cas particulier où Alk représente un groupe 1,2–propynyle, les acides (I) s'obtiennent par carbonatation de la pyridazine substituée en position 3 par un groupe –NH–Alk où Alk est un groupe 1,2–propynyle.

L'invention concerne également les dérivés de la pyridazine actifs sur le système nerveux central, obtenus par le procédé qui comprend les étapes de:

1) réaction d'une pyridazine chlorée en position 3, répondant à la formule *1*:

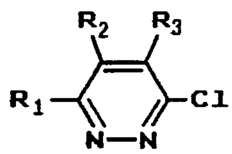

1

dans laquelle:

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$, un groupe phényle;

$R_3$ représente l'hydrogène, un groupe alkyle en $C_1$–$C_4$, un groupe phényle;

avec un excès d'hydrate d'hydrazine $NH_2$–$NH_2$ pour former la pyridazine de formule *2*:

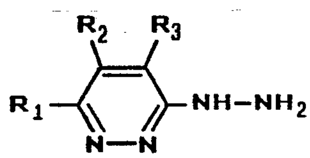

2

dans laquelle $R_1$, $R_2$ et $R_3$ sont tels que définis ci-dessus;

2) transformation de la pyridazine de formule *2* en l'amino– pyridazine correspondante de formule *3* par hydrogénation catalytique:

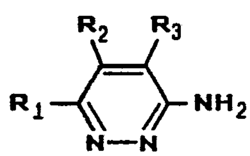

3

dans laquelle $R_1$, $R_2$ et $R_3$ sont tels que définis ci-dessus;

3) réaction de l'amino–3 pyridazine avec un composé ω–halogéné de formule X–Alk–$R_4$ dans laquelle X est un atome d'halogène et $R_4$ est un groupe COO–alkyle en $C_1$–$C_4$ ou le groupe cyano;

4) transformation éventuelle du composé ainsi obtenu par saponification ou par action de l'ammoniac dans un alcool aliphatique;

5) transformation éventuelle des dérivés obtenus en l'un de leurs sels d'addition avec des acides.

L'invention concerne tout particulièrement les dérivés de la pyridazine obtenus selon le procédé décrit ci-dessus en utilisant dans l'étape 1) une pyridazine de formule *2* dans laquelle $R_1$ est 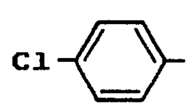

$R_2$ et $R_3$ sont l'hydrogène ou bien $R_1$ est 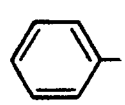 ,

$R_2$ est l'hydrogène et $R_3$ est le groupe méthyle, et dans l'étape 3) le composé ω–halogéné de formule X–Alk–$R_4$ dans laquelle Alk est $(CH_2)_3$ et $R_4$ est le groupe $-COOC_2H_5$, les composés résultants étant ensuite transformés par saponification en l'acide correspondant.

L'invention concerne aussi les dérivés de la pyridazine obtenus par le procédé qui consiste à faire réagir l'amino–3 pyridazine de formule 3 avec un halogénure X–Alk dans laquelle Alk est un groupe 1,2–propynyle et

à soumettre le produit obtenu à une carbonatation.

Les chloro–3 pyridazines utilisées comme produits de départ sont des composés connus ou peuvent être préparés selon des procédés connus et notamment par action de l'oxychlorure de phosphore en excès sur les *2H* pyridazones–3 correspondantes.

Les exemples suivants non limitatifs sont donnés à titre d'illustration de la présente invention.

**Exemple 1**

Composé obtenu à partir de la pyridazine de formule

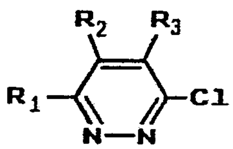

dans laquelle

(I) $R_1$ = 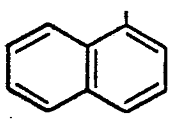 ; $R_2$ = H ; $R_3$ = $CH_3$ ;

et a partir d'un ester un halogéné de formule X–Alk–$R_4$ dans lequel Alk = $(CH_2)_3$ et $R_4$ = $-COOC_2H_5$.

**a) - Hydrazino–3 méthyl–4 (naphtyl–1)–6 pyridazine.**

On chauffe au reflux le mélange de 6,0 g de chloro–3 méthyl–4 (naphtyl–1)–6 pyridazine et 4,8 g d'hydrate d'hydrazine pendant 4 heures. Par refroidissement, il se forme un précipité que l'on essore et lave avec de l'eau. On recristallise dans le méthanol. F: 206°C.

**b) - Amino–3 méthyl–4 (naphtyl–1)–6 pyridazine.**

A la solution de 5,0 g du dérivé hydrazino obtenu ci-dessus dans le méthanol, on ajoute 2 g de nickel de Raney et on hydrogène à température ambiante sous une atmosphère pendant 72 heures. On filtre le catalyseur puis on évapore le solvant à siccité sous vide. On recristallise le résidu dans le méthanol. F: 110°C.

On dissout 1,18 g du dérivé aminé du paragraphe b) dans le minimum de diméthylformamide puis on ajoute 1,46 g de ω–bromo–butyrate d'éthyle. On chauffe à 80°C pendant 3 heures. Après refroidissement, on dilue avec de l'eau, et on alcalinise avec de la soude 1 N. On extrait avec de l'acétate d'éthyle et on sèche la phase organique sur sulfate de magnésium. On évapore à siccité sous vide. On reprend le résidu huileux dans un peu de méthanol et on fait barboter du gaz chlorhydrique dans la solution jusqu'à pH acide. On ajoute de l'éther anhydre et on essore le précipité. On recristallise dans l'isopropanol. F: 168°C.

**Exemples 2 à 26**

En opérant comme indiqué ci-dessus, mais en faisant varier les chloro–3 pyridazines

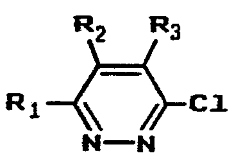

de départ et/ou les esters halogénés X–Alk–$R_4$ qui leur sont opposés, on obtient d'autres composés ci-après réunis dans le tableau I.

**Tableau I**

| Valeurs des substituants dans les réactifs pyridazine et esters ω–halogéné: | | | | | | Caractéristique physique du derivé de la pyridazène obtenu: |
|---|---|---|---|---|---|---|
| Exemple N° | $R_1$ | $R_2$ | $R_3$ | Alk | $R_4$ | Base ou sel Pt de fusion °C (solvant) |
| 2 | 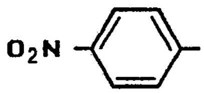 | H | H | $(CH_2)_3$ | $COOC_2H_5$ | Bromhydrate 196 (Ethanol à 95-Ether) |
| 3 | " " | H | $CH_3$ | " | " | Bromhydrate (1) |
| 4 | 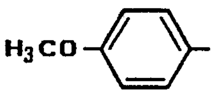 | H | $CH_3$ | " | " | Bromhydrate > 250 (Diméthylformamide) |
| 5 | 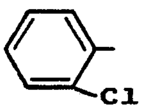 | H | H | " | " | Bromhydrate 250 (Isopropanol) |
| 6 | " " | H | $CH_3$ | " | " | Bromhydrate (1) |
| 7 | 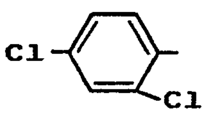 | H | H | " | " | Bromhydrate 220 (Isopropanol) |
| 8 | 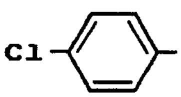 | H | H | " | " | Bromhydrate 250 (précipité) |
| 9 | 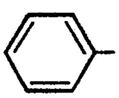 | $CH_3$ | H | " | " | Bromhydrate 140 (Acétate d'éthyle) |
| 10 | 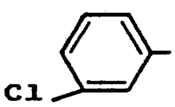 | H | $CH_3$ | " | " | Bromhydrate (1) |
| 11 | 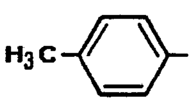 | H | $CH_3$ | " | " | Bromhydrate 260 |
| 12 | 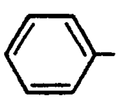 | H | CN | " | $COOCH_3$ | Base 152 * (chromatographié) |
| 13 | 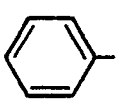 | H | 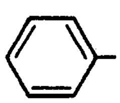 | " | $COOC_2H_5$ | Chlorhydrate 204 (Isopropanol-éther) |
| 14 | 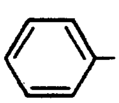 | H | $CH_3$ | " | " | Chlorhydrate 248 |

* Dans le cas de l'exemple 12 le produit obtenu à la formule:

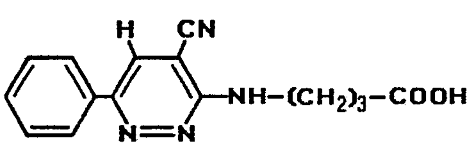

**Tableau I** (suite)

| | | | | | | |
|---|---|---|---|---|---|---|
| 15 | 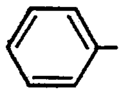 | H | H | " | " | Chlorhydrate 245 |
| 16 | " | H | $CH_3$ | $(CH_2)_2$ | " | Bromhydrate (1) |
| 17 | " | H | $CH_3$ | $(CH_2)_4$ | " | Bromhydrate (1) |
| 18 | 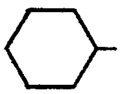 | H | H | $(CH_2)_3$ | " | Bromhydrate 149 (Isopropanol-éther) |
| 19 | 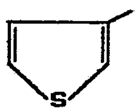 | H | H | " | " | Bromhydrate 243 (Ethanol absolu) |
| 20 | 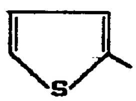 | H | H | " | " | Bromhydrate 250 (précipité) |
| 21 | $CH_3$ | 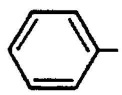 | H | " | " | Bromhydrate 174 (Isopropanol) |
| 22 | H | 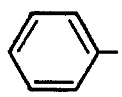 | H | " | " | Bromhydrate (1) |
| 23 | 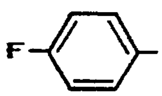 | H | H | " | " | Bromhydrate (1) |

(1) utilisé tel quel pour saponification en acide

**Exemple 24**

On dissout 1,8 g de l'ester obtenu à l'exemple 1 dans un mélange de 81 ml d'acide acétique et 9 ml d'acide chlorhydrique concentré. On chauffe le mélange à 100°C pendant 9 heures puis on évapore à siccité sous vide. Le résidu solide est recristallisé dans l'isopropanol et fournit le produit correspondant dans lequel $R_4$ a été transformé en groupe COOH.

F : 260°C

**Exemples 25 à 46**

En opérant comme dans l'exemple 24 à partir des différents esters de l'exemple 1, on obtient les acides correspondant dont les caractéristiques physiques et éventuellement la structure figurent dans le tableau II ci-après.

F : 260°C

**Tableau II**

| Exemple N° | R1 | R2 | R3 | Alk | Sel Pt de fusion (°C) (solvant) |
|---|---|---|---|---|---|
| | Valeurs des substituants dans les réactifs utilisés pour la préparation des esters à transformer | | | | Caractéristiques physiques de l'acide obtenu |
| 25 | 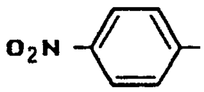 | H | H | $(CH_2)_3$ | Bromhydrate 204 (Ethanol-éther) |
| 26 | " " | H | $CH_3$ | " | Chlorhydrate 258 |
| 27 | 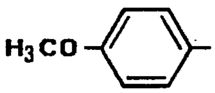 | H | $CH_3$ | " | Bromhydrate 226 (prépicité) |
| 28 | 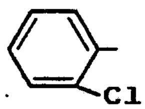 | H | H | " | Bromhydrate 226 (Isopropanol) |
| 29 | " " | H | $CH_3$ | " | Chlorhydrate 0,5 $H_2O$ 221 (Ethanol-éther) |
| 30 | 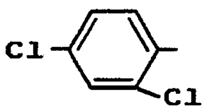 | H | H | " | Chlorhydrate > 260 |
| 31 | 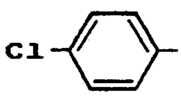 | H | H | " | Bromhydrate 250 (Isopropanol-eau) |
| 32 | 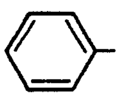 | $CH_3$ | H | " | Bromhydrate 0,25 $H_2O$ 165 (Isopropanol-éther isopropylique) |
| 33 | 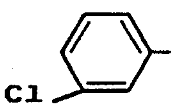 | H | $CH_3$ | " | Bromhydrate 235 (précipité) |
| 34 | 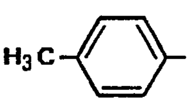 | H | $CH_3$ | " | Bromhydrate 264 (isopropanol-eau) |
| 35 | 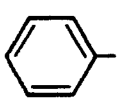 | H | CN | " | Chlorhydrate 0,5 $H_2O$ (précipité) * |
| 36 | " | H | 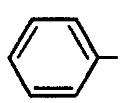 | " | Chlorhydrate 204 (Isopropanol-éther) |

* Dans le cas de l'exemple 12 le produit obtenu à la formule:

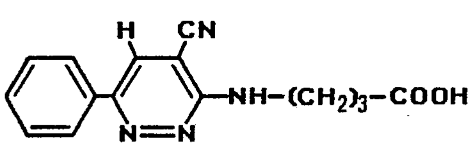

**Tableau II** (suite)

| | | | | | |
|---|---|---|---|---|---|
| 37 | " | H | $CH_3$ | $(CH_2)_3$ | Chlorhydrate 238 (Acide acétique-éther) |
| 38 | " | H | H | " | Chlorhydrate 240 |
| 39 | " | H | $CH_3$ | $(CH_2)_2$ | Bromhydrate 210 (Acide acétique-éther) |
| 40 | " | H | $CH_3$ | $(CH_2)_4$ | Chlorhydrate 236 |
| 41 | 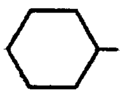 | H | H | $(CH_2)_3$ | Bromhydrate 170 (Isopropanol-éther) |
| 42 | 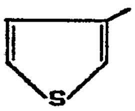 | H | H | " | Bromhydrate 252 (Ethanol 95-éther) |
| 43 | 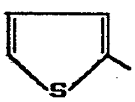 | H | H | " | Bromhydrate 228 (Isopropanol-eau) |
| 44 | $CH_3$ | 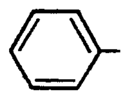 | H | " | Bromhydrate 186 (Isopropanol-éther) |
| 45 | H | 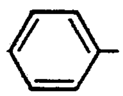 | H | " | Chlorhydrate 238 |
| 46 | 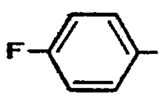 | H | H | " | Chlorhydrate 240 |

**Exemple 47**

On chauffe à reflux pendant 15 heures une solution de 2 g de l'acide de l'exemple 27 dans 20 ml d'acide bromhydrique à 48 %. Après refroidissement, on essore le solide qui s'est formé, qu'on lave avec de l'isopropanol puis avec de l'éther.

On sèche le solide à 70°C sous vide pour obtenir 2 g du produit attendu. F > 260°C.

**Exemple 48**

On prépare la méthyl–4 phényl–6 (propyne–2 ylamino)–3 pyridazine par chauffage à 60°C pendant 2 heures de 7 g d'amino–3 méthyl–4 phényl–6 pyridazine et 9 ml de bromure de propargyle. Après évaporation de l'excès de bromure de propargyle, on reprend le résidu dans 300 ml de benzène anhydre et ajoute 1,77 g de sodium. On porte au reflux pendant 15 heures, puis on verse la solution sur du carboglace en excès et on laisse en contact pendant plusieurs heures.

On évapore le solvant, reprend le résidu dans l'isopropanol et fait passer un courant de gaz chlorhydrique. On essore le solide et recristallise 2 fois dans l'isopropanol. F: 101°C.

**Exemple 49**

On dissout 1,3 g de l'ester de l'exemple 8 sous forme de base dans 100 ml de méthanol puis on refroidit la solution dans la glace et dissout 13 g d'ammoniac dans la solution. On laisse 6 jours sous agitation à température ambiante puis on évapore à siccité sous vide. On recristallise le résidu dans l'acétonitrile. On obtient ainsi le produit attendu (0,7 g). F: 170 puis 180°C.

**Exemple 50**

On dissout 2,47 g d'amino-3 diphényl-4,6 pyridazine dans 5 ml de diméthylformamide et on ajoute 1,63 g de bromo-4 butyronitrile. On chauffe à 60°C pendant 2 heures et on laisse refroidir. On essore les cristaux formés et on recristallise dans l'isopropanol. F = 202 – 204°C.

**Exemples 51 et 52**

En opérant de la même façon, mais en faisant varier l'amino-3 pyridazine de départ, on obtient de la même façon:
– à partir de l'amino-3 methyl-4 phenyl-6 pyridazine on obtient un produit ayant un point de fusion F > 265°C
– et à partir de l'amino-3 phenyl-6 pyridazine on obtient un produit ayant un point de fusion F: 265 – 264°C.

**Exemple 53**

On dissout 3,33 g de bromhydrate du nitrile de l'exemple 51 dans 100 ml d'acide formique sec puis, en agitant, on fait barboter pendant 4 heures un courant d'acide chlorhydrique gazeux sec avec un débit d'environ 5 litres/heure. On évapore à siccité sous vide en chauffant le moins possible. Le résidu est repris dans l'éthanol et on ajoute de l'éther anhydre. On essore les cristaux et on recristallise dans l'isopropanol. F: 130 – 132°C.

Les produits selon l'invention ont été étudiés en ce qui concerne leur activité sur le système nerveux central. Activité sur le déplacement de l'acide γ-aminobutyrique de son récepteur post-synaptique.

**Méthode**

L'activité neurochimique des dérivés de la présente invention sur le système GABA-ergique a été évaluée par la mesure du déplacement de l'acide γ-aminobutyrique (GABA) de son récepteur post-synaptique.

L'étude a été réalisée selon la méthode de ENNA et SNYDER (Brain Res. *100*, 81-97, 1975).

L'expérience de déplacement s'est effectuée in vitro en présence de suspension de membranes synaptiques et de GABA tritié à la concentration finale de 3,6 nM.

**Résultats**

| Produit de l'exemple n° | Concentrations efficaces médianes ($CE_{50}$) de déplacements du GABA tritié en µM |
|---|---|
| 37 | 2,8 |
| 38 | 1,34 |
| 24 | 2,9 |
| 39 | 10 |
| 33 | 0,70 |
| 31 | 1,25 |
| 27 | 5,0 |
| 42 | 2,4 |
| 41 | 10,5 |
| 47 | 3,0 |
| 43 | 0,83 |
| 30 | 7,0 |
| 29 | 3,2 |
| 34 | 0,71 |

**Commentaires**

Les produits de l'invention présentent l'aptitude de déplacer le GABA de son récepteur synaptique.

Cette étude in vitro a été complétée par une étude in vivo.

Les tests suivants ont été utilisés.

**1 - Activité sur la motilité de la souris.**

**Méthode**

L'activité tranquillisante-sédative des dérivés de la présente invention a été évaluée par la mesure de la motilité spontanée des souris grâce au test d'actimétrie (J.R. BOISSIER et P. SIMON, Arch Int Pharmacocyn, 1965, *158*, 212-221).

L'équipement était composé de cages actimétriques type Apelab (L = 26 cm; 1 = 21,5 cm; H = 10 cm) traversées par deux rayons lumineux qui impressionnent une cellule photoélectrique.

Les animaux ont été placés individuellement dans les cages 45 minutes après l'administration du produit par voie orale: chaque traversée d'un faisceau lumineux a été comptabilisée par un compteur individuel. Les scores correspondant aux déplacements des animaux ont été enregistrés pendant 10 minutes. Les lots étaient constitués de 12 souris par dose.

**2 - Effet sur l'antagonisme de la ptôse induite par la réserpine.**

**Méthode**

L'activité antidépressive des composés a été évaluée dans le test de l'antagonisme de la ptôse à la réserpine chez la souris.

Cette étude a été effectuée sur des lots de 10 souris femelles de 20 ± 1 g. Les produits ont été administrés par voie i.p. en même temps que la réserpine (2 mg/kg, i.v.). Les souris ont été observées individuellement 1 heure après les administrations. Les animaux qui n'ont pas présenté de ptôse pendant les 15 secondes de l'observation ont été considérés comme antagonisés. Tous les animaux témoins n'ayant reçu que le véhicule et la réserpine ont présenté la ptôse. La dose efficace médiane d'antagonisme ($DE_{50}$) a été évaluée par la méthode des probits.

**3 - Effet sur le comportement rotatoire chez la souris après lésion unilatérale de la voie nigro-striée par la 6-(OH) dopamine.**

**Méthode**

L'influence des dérivés de la présente invention sur le système dopaminergique central a été évaluée sur le comportement rotatoire chez la souris après lésion unilatérale de la voie nigro-striée par la 6-(OH) dopamine (P. PROTAIS et J. COSTENTIN, J. Pharmacol, *7* (2), 251-255, 1976).

Des souris femelles Charles River CD1, pesant 20 à 24 g, ont préalablement fait l'objet d'une lésion unilatérale du striatum par injection stéréotaxique de 6-(OH) dopamine à raison de 8 µg par animal. Une semaine après cette opération, le produit à étudier a été administré par voie intrapéritonéale à des groupes de 7 souris. Le nombre de rotations a été évalué pendant 2 minutes, 1 heure après l'administration du produit. Les rotations ipsilatérales à la lésion ont été comptées positivement, celles contralatérales ont été comptées négativement. La somme algébrique des rotations pour un groupe d'animaux traités a été comparée à celle du groupe d'animaux témoins n'ayant reçu que le véhicule (sérum physiologique).

Les résultats obtenus avec l'un des produits représentatifs de l'invention, à savoir le produit de l'exemple n° 40, figurent dans le tableau III ci-après.

**Tableau III**

| Motilité de la souris | | Antagonisme de la ptôse à la réserpine | Comportement rotatoire chez la souris | |
|---|---|---|---|---|
| Dose en mg/kg | Effet | $DE_{50}$ mg/kg | Dose en mg/kg | Effet |
| 100 | -48 %** | 29 | 0,5 | -50 %** |
| 50 | -33 %* | (26 - 32)* | 2 | -62 %** |

* : p 0,05
**: p 0,01

Les essais ainsi effectués montrent que les produits selon l'invention agissent sur le neurone par occupation du site récepteur de l'acide γ-aminobutyrique. Ils présentent des propriétés pharmacologiques chez l'animal pouvant les conduire à être utilisés en thérapeutique humaine pour le traitement des affections psychiques neurologiques ou neuromusculaires.

En particulier les produits selon l'invention peuvent être utilisés pour les troubles de l'humeur ou du comportement tels que les états dépressifs, l'asthénie, la maladie de Parkinson, les troubles du comportement alimentaire ou l'insomnie.

Ces produits peuvent être administrés par voie orale ou par voie injectable. Les compositions pharmaceutiques peuvent être solides ou liquides et se présenter par exemple sous forme de comprimés, gélules, granulés, suppositoires ou préparations injectables.

La posologie peut varier dans de larges proportions, en particulier suivant le type et la gravité de l'affection à traiter et suivant le mode d'administration. Le plus souvent chez l'adulte par voie orale, elle est comprise entre 0,050 et 0,500 g par jour éventuellement répartie en plusieurs prises.

A titre d'exemple, on peut indiquer la préparation galénique suivante:

**Gélules**

| | |
|---|---|
| Produit de l'exemple n° 40 | 100 mg |
| Aérosil | 0,5 mg |
| Stéarate de magnésium | 1,5 mg |
| Amidon STA RX 1500 | 40 mg |
| | 150 mg |

**Revendication** pour les Etats contractants: BE, CH, DE, FR, GB, IT, LI, LU, NL, SE.

1. Dérivés de la pyridazine amino-substitués en position 3, caractérisés en ce qu'ils répondent à la formule générale:

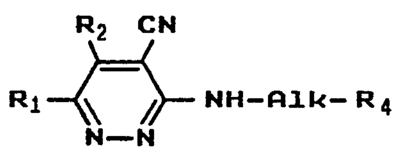

(I)

dans laquelle:

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$, un groupe phényle;

Alk représente un groupe $(CH_2)_n$ où n est un entier égal à 2, 3, 4 ou encore un groupe 1,2–propynyle $-CH_2C{\equiv}C-$;

$R_4$ représente: –COOH
–COO–alkyle en $C_1$–$C_4$
$-CONH_2$
$-C{\equiv}N$

et les sels d'addition desdits dérivés avec des acides.

2. Dérivé de la pyridazine selon la revendication 1, caractérisé en ce que:

$R_1$ est le groupe 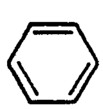

$R_2$ est l'hydrogène,
Alk est le groupe $-(CH_2)_3-$
$R_4$ est le groupe $-COOCH_3$.

3. Dérivé de la pyridazine selon la revendication 1, caractérisé en ce que:

$R_1$ est le groupe 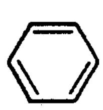

$R_2$ est l'hydrogène,
Alk est le groupe $-(CH_2)_3-$ et
$R_4$ est le groupe –COOH.

4. Procédé pour l'obtention des dérivés de pyridazine de formule (I) dans lesquels Alk est un groupe $(CH_2)_n$, selon la revendication 1, caractérisé en ce qu'il consiste:

1) à faire réagir une pyridazine chlorée en position 3 de formule *1*:

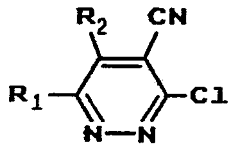

*1*

avec un excès d'hydrate d'hydrazine $NH_2$–$NH_2$ pour former la pyridazine de formule *2*:

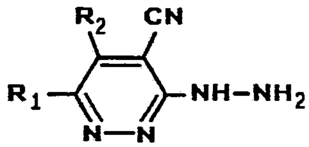

*2*

2) à transformer la pyridazine de formule *2* en l'amino–3 pyridazine correspondante par hydrogénation catalytique;

3) à faire réagir ladite amino–3 pyridazine avec un composé ω–halogéné de formule X–Alk–$R_4$ dans laquelle X est un atome d'halogène et $R_4$ est un groupe COO–alkyle en $C_1$–$C_4$ ou le groupe cyano pour fournir le composé de formule (I) dans laquelle $R_4$ est tel que défini ci-dessus;

4) à transformer éventuellement le composé de formule (I) ainsi obtenu en un composé de formule (I) dans laquelle $R_4$ est le groupe COOH ou $CONH_2$; et

5) à transformer éventuellement les dérivés obtenus en des sels d'addition avec des acides.

5. Procédé selon la revendication 4, caractérisé en ce que l'étape 4) consiste en une saponification du composé de formule (I) dans laquelle $R_4$ est le groupe COO–alkyle en $C_1$–$C_4$ pour former l'acide correspondant, à savoir le composé de formule (I) dans laquelle $R_4$ est –COOH.

6. Procédé selon la revendication 5, caractérisé en ce que la saponification est effectuée en milieu acide par des moyens connus.

7. Procédé selon la revendication 6, caractérisé en ce que la saponification est effectuée par chauffage avec un hydracide, au sein de l'acide acétique à une température comprise entre 20 et 100°C.

8. Procédé selon la revendication 7, caractérisé en ce que l'hydracide est l'acide chlorhydrique ou l'acide bromhydrique.

9. Procédé selon la revendication 4, caractérisé en ce que l'étape 4) consiste en la transformation du composé de formule (I) dans laquelle $R_4$ est le groupe COO–alkyle en $C_1$–$C_4$ ou le groupe cyano en le composé de formule (I) dans laquelle $R_4$ est un groupe carboxamido.

10. Procédé pour l'obtention des dérivés de pyridazine de formule (I) dans laquelle $R_1$, $R_2$ et $R_4$ sont tels que définis dans la revendication 1, et Alk est un groupe 1,2–propynyle, caractérisé en ce qu'il consiste à soumettre à une carbonatation la pyridazine correspondante substituée en position 3 par le groupe –NH–Alk dans laquelle Alk est un groupe 1,2–propynyle.

11. Dérivés de la pyridazine actifs sur le système nerveux central, caractérisé en ce qu'ils sont obtenus par le procédé qui comprend les étapes de:

1) réaction d'une pyridazine chlorée en position 3, répondant à la formule *1*:

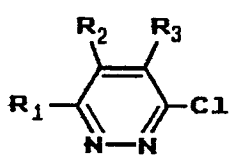

*1*

dans laquelle:

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$, un groupe phényle;
$R_3$ représente l'hydrogène, un groupe alkyle en $C_1$–$C_4$, un groupe phényle;

avec un excès d'hydrate d'hydrazine $NH_2$–$NH_2$ pour former la pyridazine de formule *2*:

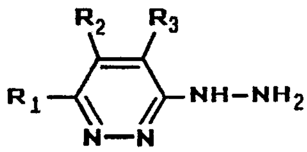

*2*

dans laquelle $R_1$, $R_2$ et $R_3$ sont tels que définis ci-dessus;

2) transformation de la pyridazine de formule *2* en l'amino–3 pyridazine correspondante de formule *3* par hydrogénation catalytique:

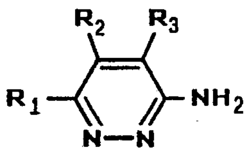

*3*

dans laquelle $R_1$, $R_2$ et $R_3$ sont tels que définis ci-dessus;

3) réaction de l'amino–3 pyridazine avec un composé ω–halogéné de formule X–Alk–$R_4$ dans laquelle X est un atome d'halogène et $R_4$ est un groupe COO–alkyle en $C_1$–$C_4$ ou le groupe cyano;

4) transformation éventuelle du composé ainsi obtenu par saponification ou par action de l'ammoniac dans un alcool aliphatique;

5) transformation éventuelle des dérivés obtenus en l'un de leurs sels d'addition avec des acides.

12. Dérivé selon la revendication 11, caractérisé en ce que dans l'étape 1) on utilise la pyridazine de formule *2* dans laquelle

$R_1$ est le radical

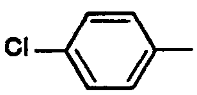

$R_2$ et $R_3$ sont l'hydrogène et en ce que dans l'étape 3) on utilise le composé ω–halogéné de formule X–Alk–$R_4$ dans laquelle Alk est le groupe $-(CH_2)_3-$; X est un halogène et $R_4$ est le groupe $-COOC_2H_5$ et en ce que l'on transforme le composé ainsi obtenu par saponification en l'acide correspondant.

13. Dérivé selon la revendication 11, caractérisé en ce que dans l'étape 1) on utilise la pyridazine de formule *2* dans laquelle

$R_1$ est le radical

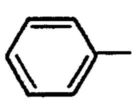

$R_2$ est l'hydrogène et
$R_3$ est le groupe méthyle,

en ce que dans l'étape 3 on utilise le composé ω–halogéné de formule X–Alk–$R_4$ dans laquelle Alk est le groupe $-(CH_2)_3-$; X est un halogène et $R_4$ est le groupe $-COOC_2H_5$ et en ce que l'on transforme le composé ainsi obtenu par saponification en l'acide correspondant.

14. Dérivés de la pyridazine, caractérisés en ce qu'ils sont obtenus par le procédé qui consiste à faire réagir l'amino–3 pyridazine de formule *3* avec un halogénure X–Alk dans laquelle Alk est un groupe 1,2–propynyle et à soumettre le produit obtenu à une carbonatation.

15. Médicaments caractérisés en ce qu'ils contiennent un des dérivés pharmaceutiquement acceptables de la pyridazine selon l'une quelconque des revendications 1 à 3 et 11 à 14.

16. Médicaments selon la revendication 15, caractérisés en ce qu'ils sont actifs sur le système nerveux central.

17. Médicaments selon l'une des revendications 15 ou 16, caractérisés en ce qu'ils sont conditionnés en vue d'une administration par voie orale ou par voie injectable.

**Revendications** pour l'Etat contractant: AT

1. Procédé pour l'obtention de dérivés de la pyridazine amino-substitués en position 3, répondant à la formule générale:

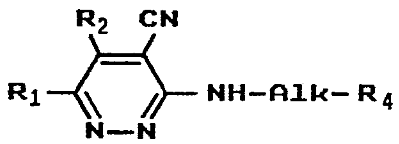

(I)

dans laquelle:

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$, un groupe phényle;

Alk représente un groupe $(CH_2)_n$ où n est un entier égal à 2, 3, 4;

$R_4$ représente: –COOH

–COO–alkyle en $C_1$–$C_4$

–$CONH_2$

–C≡N

et les sels d'addition desdits dérivés avec des acides, caractérisé en ce qu'il consiste:

1) à faire réagir une pyridazine chlorée en position 3 de formule *1*:

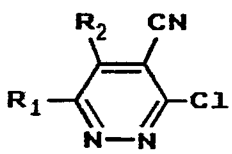

*1*

avec un excès d'hydrate d'hydrazine $NH_2$–$NH_2$ pour former la pyridazine de formule *2*:

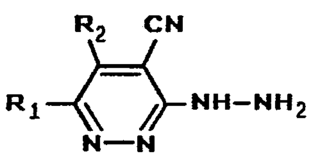

*2*

2) à transformer la pyridazine de formule *2* en l'amino–3 pyridazine correspondante par hydrogénation catalytique;

3) à faire réagir ladite amino–3 pyridazine avec un composé ω–halogéné de formule X–Alk–$R_4$ dans laquelle X est un atome d'halogène et $R_4$ est un groupe COO–alkyle en $C_1$–$C_4$ ou le groupe cyano pour fournir le composé de formule (I) dans laquelle $R_4$ est tel que défini ci-dessus;

4) à transformer éventuellement le composé de formule (I) ainsi obtenu en un composé de formule (I) dans laquelle $R_4$ est le groupe COOH ou $CONH_2$; et

5) à transformer éventuellement les dérivés obtenus en des sels d'addition avec des acides.

2. Procédé selon la revendication 1, caractérisé en ce que l'étape 4) consiste en une saponification du composé de formule (I) dans laquelle $R_4$ est le groupe COO–alkyle en $C_1$–$C_4$ pour former l'acide correspondant, à savoir le composé de formule (I) dans laquelle $R_4$ est –COOH.

3. Procédé selon la revendication 2, caractérisé en ce que la saponification est effectuée en milieu acide par des moyens connus.

4. Procédé selon la revendication 3, caractérisé en ce que la saponification est effectuée par chauffage avec un hydracide, au sein de l'acide acétique à une température comprise entre 20 et 100°C.

5. Procédé selon la revendication 4, caractérisé en ce que l'hydracide est l'acide chlorhydrique ou l'acide bromhydrique.

6. Procédé selon la revendication 1, caractérisé en ce que l'étape 4) consiste en la transformation du composé de formule (I) dans laquelle $R_4$ est le groupe COO–alkyle en $C_1$–$C_4$ ou le groupe cyano en le composé de formule (I) dans laquelle $R_4$ est un groupe carboxamido.

7. Procédé pour l'obtention de dérivés de pyridazine de formule (I) dans laquelle $R_1$, $R_2$ et $R_4$ sont tels que définis dans la revendication 1, et Alk est un groupe 1,2–propynyle, caractérisé en ce qu'il consiste à soumettre à une carbonatation la pyridazine correspondante substituée en position 3 par le groupe –NH–Alk dans laquelle Alk est un groupe 1,2–propynyle.

8. Procédé selon l'une quelconque des revendications 1 à 6, caractérisé en ce que l'on utilise un composé de formule 1 dans laquelle $R_1$ est le groupe

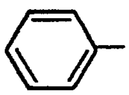

et $R_2$ est l'hydrogène, et en ce que l'on utilise dans l'étape 3) un composé ω–halogéné de formule X–Alk–$R_4$ dans laquelle X est un atome d'halogène, Alk est le groupe $-(CH_2)_3-$ et $R_4$ est le groupe $-COOCH_3$.

9. Procédé selon l'une quelconque des revendications 1 à 6, caractérisé en ce que l'on utilise un composé de formule 1 dans laquelle $R_1$ est le groupe

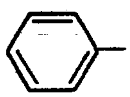

et $R_2$ est l'hydrogène, et en ce que l'on utilise dans l'étape 3) un composé ω–halogéné de formule X–Alk–$R_4$ dans laquelle X est un atome d'halogène, Alk est le groupe $-(CH_2)_3$ et $R_4$ est le groupe $-COOH$.

10. Procédé pour l'obtention de dérivés de la pyridazine actifs sur le système nerveux central, caractérisé en ce qu'il comprend les étapes de:

1) réaction d'une pyridazine chlorée en position 3, répondant à la formule *1*:

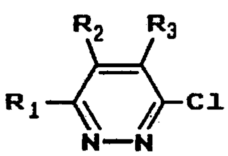

*1*

dans laquelle:

$R_1$ désigne l'hydrogène, un groupe alkyle en $C_1$–$C_4$, un groupe phényle, un groupe phényle monosubstitué par un halogène ou un groupe nitro ou un groupe alkyle en $C_1$–$C_4$ ou un groupe alcoxy en $C_1$–$C_4$ ou un groupe hydroxy ou un groupe trifluorométhyle, un groupe phényle disubstitué par un halogène, un groupe naphtyle, un groupe cyclohexyle, un groupe thiényle–2, un groupe thiényle–3 ou un groupe indolyle–3;

$R_2$ représente l'hydrogène, un groupe alkyle en $C_1$–$C_4$, un groupe phényle;

$R_3$ représente l'hydrogène, un groupe alkyle en $C_1$–$C_4$, un groupe phényle;

avec un excès d'hydrate d'hydrazine $NH_2$–$NH_2$ pour former la pyridazine de formule *2*:

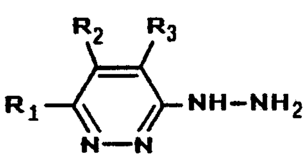

*2*

dans laquelle $R_1$, $R_2$ et $R_3$ sont tels que définis ci-dessus;

2) transformation de la pyridazine de formule *2* en l'amino–3 pyridazine correspondante de formule *3* par hydrogénation catalytique:

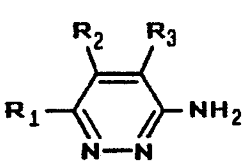

*3*

dans laquelle $R_1$, $R_2$ et $R_3$ sont tels que définis ci-dessus;

3) réaction de l'amino-3 pyridazine avec un composé ω-halogéné de formule X-Alk-$R_4$ dans laquelle X est un atome d'halogène et $R_4$ est un groupe COO-alkyle en $C_1$-$C_4$ ou le groupe cyano;

4) transformation éventuelle du composé ainsi obtenu par saponification ou par action de l'ammoniac dans un alcool aliphatique.

5) transformation éventuelle des dérivés obtenus en l'un de leurs sels d'addition avec des acides.

11. Procédé selon la revendication 10, caractérisé en ce que dans l'étape 1) on utilise la pyridazine de formule *2* dans laquelle

$R_1$ est le radical

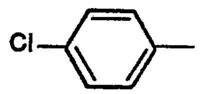

$R_2$ et $R_3$ sont l'hydrogène et en ce que dans l'étape 3) on utilise le composé ω-halogéné de formule X-Alk-$R_4$ dans laquelle Alk est le groupe $-(CH_2)_3-$; X est un halogène et $R_4$ est le groupe $-COOC_2H_5$ et en ce que l'on transforme le composé ainsi obtenu par saponification en l'acide correspondant.

12. Procédé selon la revendication 10, caractérisé en ce que dans l'étape 1) on utilise la pyridazine de formule *2* dans laquelle

$R_1$ est le radical

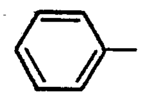

$R_2$ est l'hydrogène et

$R_3$ est le groupe méthyle,

en ce que dans l'étape 3 on utilise le composé ω-halogéné de formule X-Alk-$R_4$ dans laquelle Alk est le groupe $-(CH_2)_3-$; X est un halogène et $R_4$ est le groupe $-COOC_2H_5$ et en ce que l'on transforme le composé ainsi obtenu par saponification en l'acide correspondant.

13. Procédé pour l'obtention de dérivés de la pyridazine actifs sur le système nerveux central, caractérisé en ce qu'il consiste faire réagir l'amino-3 pyridazine de formule *3* avec un halogénure X-Alk dans laquelle Alk est un groupe 1,2-propynyle et à soumettre le produit obtenu à une carbonatation.

14. Utilisation des composés obtenus par le procédé selon l'une quelconque des revendications 1 à 13 pour la fabrication de médicaments.

15. Utilisation des composés obtenus par le procédé selon l'une quelconque des revendications 1 à 13, pour la fabrication de médicaments actifs sur le système nerveux central.

16. Utilisation selon l'une des revendications 14 ou 15, pour la fabrication de médicaments conditionnés en vue d'une administration par voie orale ou par voie injectable.

**Patentansprüche** für die Vertragsstaaten: BE, CH, DE, FR, GB, IT, LU, NL, SE

1. In Position 3 aminosubstituierte Pyridazinderivate, dadurch gekennzeichnet, daß sie der allgemeinen Formel

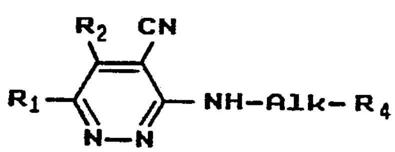

(I)

entsprechen, worin:

$R_1$ Wasserstoff, eine $C_1$-$C_4$-Alkylgruppe, eine Phenylgruppe, eine durch ein Halogen oder eine Nitrogruppe oder eine $C_1$-$C_4$-Alkylgruppe oder eine $C_1$-$C_4$-Alkoxygruppe oder eine Hydroxygruppe oder eine Trifluormethylgruppe monosubstituierte Phenylgruppe, eine durch ein Halogen disubstituierte Phenylgruppe, eine Naphthylgruppe, eine Cyclohexylgruppe, eine 2-Thienylgruppe, eine 3-Thienylgruppe oder eine 3-Indolylgruppe bezeichnet;

$R_2$ Wasserstoff, eine $C_1$–$C_4$–Alkylgruppe, eine Phenylgruppe darstellt;
Alk eine Gruppe $(CH_2)_n$ darstellt, worin n eine ganze Zahl gleich 2, 3, 4 oder auch eine 1,2–Propynylgruppe $-CH_2C{\equiv}C-$ ist;
$R_4$ für
– COOH
– $C_1$–$C_4$–COO–Alkyl
– $CONH_2$
– $C{\equiv}N$
steht;
und die Additionssalze dieser Derivate mit Säuren.

2. Pyridazinderivat nach Anspruch 1, dadurch gekennzeichnet, daß

$R_1$ die Gruppe 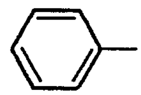 ist,

$R_2$ Wasserstoff ist,
Alk die Gruppe $-(CH_2)_3-$ ist,
$R_4$ die Gruppe $-COOCH_3$ ist.

3. Pyridazinderivat nach Anspruch 1, dadurch gekennzeichnet, daß

$R_1$ die Gruppe 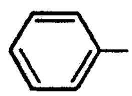 ist,

$R_2$ Wasserstoff ist,
Alk die Gruppe $-(CH_2)_3$ ist und
$R_4$ die Gruppe $-COOH$ ist.

4. Verfahren zur Herstellung von Pyridazinderivaten der Formel (I) nach Anspruch 1, worin Alk eine Gruppe $(CH_2)_n$ ist, dadurch gekennzeichnet, daß es darin besteht, daß:
1) ein in Position 3 chloriertes Pyridazin der Formel 1:

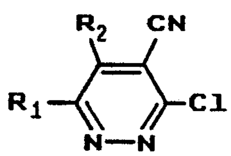

1

mit einem Überschuß an Hydrazinhydrat $NH_2$–$NH_2$ reagieren gelassen wird, um das Pyridazin der Formel 2:

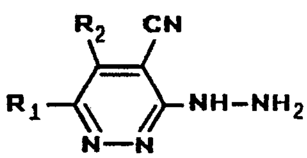

2

zu bilden;
2) das Pyridazin der Formel 2 durch katalytische Hydrierung in das entsprechende 3–Aminopyridazin übergeführt wird;
3) das 3–Aminopyridazin mit einer ω–halogenierten Verbindung der Formel X–Alk–$R_4$, worin X ein Halogenatom ist und $R_4$ eine $C_1$–$C_4$–COO–Alkylgruppe oder die Zyanogruppe darstellt, reagieren gelassen wird, um die Verbindung der Formel (I) zu liefern, worin $R_4$ wie oben definiert ist;
4) gegebenenfalls die so erhaltene Verbindung der Formel (I) in eine Verbindung der Formel (I) übergeführt wird, worin $R_4$ die Gruppe COOH oder $CONH_2$ ist; und
5) gegebenenfalls die erhaltenen Derivate in Additionssalze mit Säuren übergeführt werden.

5. Verfahren nach Anspruch 4, dadurch gekennzeichnet, daß die Stufe 4) in einer Verseifung der Verbindung der Formel (I), worin $R_4$ die Gruppe $C_1$–$C_4$–COO–Alkyl ist, besteht, um die entsprechende Säure, nämlich die Verbindung der Formel (I), worin $R_4$ für –COOH steht, zu bilden.

6. Verfahren nach Anspruch 5, dadurch gekennzeichnet, daß die Verseifung in saurem Milieu mittels bekannter Mittel durchgeführt wird.

7. Verfahren nach Anspruch 6, dadurch gekennzeichnet, daß die Verseifung durch Erhitzen mit einer Wasserstoffsäure in Essigsäure bei einer Temperatur zwischen 20 und 100°C erfolgt.

8. Verfahren nach Anspruch 7, dadurch gekennzeichnet, daß die Wasserstoffsäure Salzsäure oder Bromwasserstoffsäure ist.

9. Verfahren nach Anspruch 4, dadurch gekennzeichnet, daß die Stufe 4) in der Überführung der Verbindung der Formel (I), worin $R_4$ die Gruppe $C_1$–$C_4$–COO–Alkyl oder die Cyanogruppe ist, in die Verbindung der Formel (I), worin $R_4$ eine Carboxamidogruppe ist, besteht.

10. Verfahren zur Herstellung von Pyridazinderivaten der Formel (I), worin $R_1$, $R_2$ und $R_4$ wie in Anspruch 1 definiert sind und Alk für eine 1,2–Propynylgruppe steht, dadurch gekennzeichnet, daß es darin besteht, daß das entsprechende, in Position 3 durch die Gruppe –NH–Alk, worin Alk eine 1,2– Propynylgruppe ist, substituierte Pyridazin einer Carbonatisierung unterzogen wird.

11. Pyridazinderivate, die auf das Zentralnervensystem aktiv sind, dadurch gekennzeichnet, daß sie erhalten werden durch ein Verfahren, welches die folgenden Schritte umfaßt:

1) Umsetzung eines in Position 3 chlorierten Pyridazins der Formel 1:

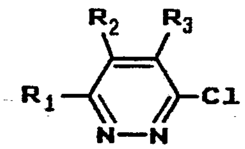

1

worin:

R1 Wasserstoff, eine $C_1$–$C_4$–Alkylgruppe, eine Phenylgruppe, eine durch ein Halogen oder eine Nitrogruppe oder eine $C_1$–$C_4$– Alkylgruppe oder eine $C_1$–$C_4$–Alkoxygruppe oder eine Hydroxygruppe oder eine Trifluormethylgruppe monosubstituierte Phenylgruppe, eine durch ein Halogen disubstituierte Phenylgruppe, eine Naphthylgruppe, eine Cyclohexylgruppe, eine 2–Thienylgruppe, eine 3–Thienylgruppe oder eine 3–Indolylgruppe bezeichnet;

$R_2$ Wasserstoff, eine $C_1$–$C_4$–Alkylgruppe, eine Phenylgruppe darstellt;

$R_3$ für Wasserstoff, eine $C_1$–$C_4$–Alkylgruppe, eine Phenylgruppe steht;

mit einem Überschuß an Hydrazinhydrat $NH_2$–$NH_2$, um das Pyridazin der Formel 2:

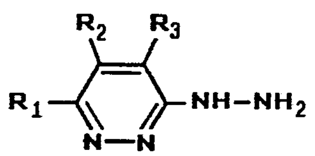

2

zu bilden, worin $R_1$, $R_2$ und $R_3$ wie oben definiert sind;

2) Überführung des Pyridazins der Formel 2 durch katalytische Hydrierung in das entsprechende 3–Aminopyridazin der Formel 3:

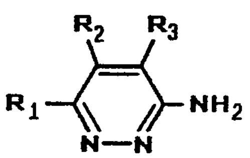

3

worin $R_1$, $R_2$ und $R_3$ wie oben definiert sind;

3) Umsetzung des 3–Aminopyridazins mit einer ω–halogenierten Verbindung der Formel X–Alk–$R_4$, worin X ein Wasserstoffatom ist und $R_4$ eine $C_1$–$C_4$–COO–Alkylgruppe oder die Cyanogruppe darstellt;

4) gegebenenfalls Überführung der so erhaltenen Verbindung durch Verseifung oder durch Einwirken von Ammoniak in einem aliphatischen Alkohol;

5) gegebenenfalls Überführung der erhaltenen Derivate in eines ihrer Additionssalze mit Säuren.

12. Derivat nach Anspruch 11, dadurch gekennzeichnet, daß man in Stufe 1) das Pyridazin der Formel 2 verwendet, worin

$R_1$ die Gruppe 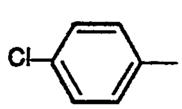 ist;

$R_2$ und $R_3$ Wasserstoff sind, und daß man in Stufe 3) die ω–halogenierte Verbindung der Formel X–Alk–$R_4$ verwendet,

worin Alk die Gruppe $-(CH_2)_3-$ ist; X ein Halogen ist und $R_4$ die Gruppe $-COOC_2H_5$ darstellt, und daß man die so erhaltene Verbindung durch Verseifung in die entsprechende Säure überführt.

13. Derivat nach Anspruch 11, dadurch gekennzeichnet, daß man in der Stufe 1) das Pyridazin der Formel 2 verwendet, worin

$R_1$ die Gruppe 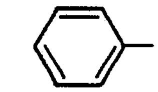 ist,

$R_2$ Wasserstoff ist und
$R_3$ für die Methylgruppe steht,

daß man in der Stufe 3) die ω-halogenierte Verbindung der Formel $X-Alk-R_4$ verwendet, worin Alk die Gruppe $-(CH_2)_3-$ ist, X ein Halogen ist und $R_4$ für die Gruppe $-COOC_2H_5$ steht, und daß man die so erhaltene Verbindung durch Verseifung in die entsprechende Säure überführt.

14. Pyridazinderivate, dadurch gekennzeichnet, daß sie erhalten werden durch ein Verfahren, welches darin besteht, daß 3-Aminopyridazin der Formel 3 mit einem Halogenid X-Alk, worin Alk eine 1,2-Propynylgruppe ist, reagieren gelassen wird und das erhaltene Produkt einer Carbonatisierung unterzogen wird.

15. Medikamente, dadurch gekennzeichnet, daß sie eines der pharmazeutisch akzeptablen Pyridazinderivate nach einem der Ansprüche 1 bis 3 und 11 bis 14 enthalten.

16. Medikamente nach Anspruch 15, dadurch gekennzeichnet, daß sie auf das Zentralnervensystem aktiv sind.

17. Medikamente nach einem der Ansprüche 15 oder 16, dadurch gekennzeichnet, daß sie zur Verabreichung auf oralem Weg oder auf Injektionsweg konditioniert sind.

**Patentansprüche** für den Vertragsstaat: AT

1. Verfahren zur Herstellung von in Position 3 aminosubstituierten Pyridazinderivaten der allgemeinen Formel

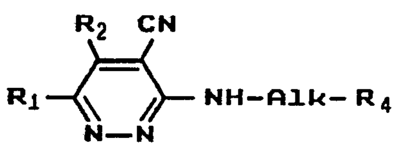

(I)

worin:

$R_1$ Wasserstoff, eine $C_1-C_4$-Alkylgruppe, eine Phenylgruppe, eine durch ein Halogen oder eine Nitrogruppe oder eine $C_1-C_4$- Alkylgruppe oder eine $C_1-C_4$-Alkoxygruppe oder eine Hydroxygruppe oder eine Trifluormethylgruppe monosubstituierte Phenylgruppe, eine durch ein Halogen disubstituierte Phenylgruppe, eine Naphthylgruppe, eine Cyclohexylgruppe, eine 2-Thienylgruppe, eine 3-Thienylgruppe oder eine 3-Indolylgruppe bezeichnet;
$R_2$ Wasserstoff, eine $C_1-C_4$-Alkylgruppe, eine Phenylgruppe darstellt;
Alk eine Gruppe $(CH_2)_n$ darstellt, worin n eine ganze Zahl gleich 2, 3, 4 ist;
$R_4$ für – COOH
– $C_1-C_4$-COO-Alkyl
– $CONH_2$
– $C\equiv N$
steht;

und der Additionssalze dieser Derivate mit Säuren, dadurch gekennzeichnet, daß es darin besteht, daß:
1) ein in Position 3 chloriertes Pyridazin der Formel 1:

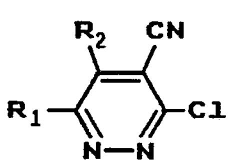

1

mit einem Überschuß an Hydrazinhydrat $NH_2-NH_2$ reagieren gelassen wird, um das Pyridazin der Formel 2:

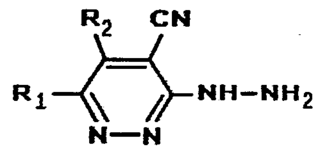

2

zu bilden;

2) das Pyridazin der Formel 2 durch katalytische Hydrierung in das entsprechende 3–Aminopyridazin übergeführt wird;

3) das 3–Aminopyridazin mit einer ω–halogenierten Verbindung der Formel X–Alk–$R_4$, worin X ein Halogenatom ist und $R_4$ eine $C_1$–$C_4$–COO–Alkylgruppe oder die Zyanogruppe darstellt, reagieren gelassen wird, um die Verbindung der Formel (I) zu liefern, worin $R_4$ wie oben definiert ist;

4) gegebenenfalls die so erhaltene Verbindung der Formel (I) in eine Verbindung der Formel (I) übergeführt wird, worin $R_4$ die Gruppe COOH oder $CONH_2$ ist; und

5) gegebenenfalls die erhaltenen Derivate in Additionssalze mit Säuren übergeführt werden.

2. Verfahren nach Anspruch 1, dadurch gekennzeichnet, daß die Stufe 4) in einer Verseifung der Verbindung der Formel (I), worin $R_4$ die Gruppe $C_1$–$C_4$–COO–Alkyl ist, besteht, um die entsprechende Säure, nämlich die Verbindung der Formel (I), worin $R_4$ für –COOH steht, zu bilden.

3. Verfahren nach Anspruch 2, dadurch gekennzeichnet, daß die Verseifung in saurem Milieu mittels bekannter Mittel durchgeführt wird.

4. Verfahren nach Anspruch 3, dadurch gekennzeichnet, daß die Verseifung durch Erhitzen mit einer Wasserstoffsäure in Essigsäure bei einer Temperatur zwischen 20 und 100°C erfolgt.

5. Verfahren nach Anspruch 4, dadurch gekennzeichnet, daß die Wasserstoffsäure Salzsäure oder Bromwasserstoffsäure ist.

6. Verfahren nach Anspruch 1, dadurch gekennzeichnet, daß die Stufe 4) in der Überführung der Verbindung der Formel (I), worin $R_4$ die Gruppe $C_1$–$C_4$–COO–Alkyl oder die Cyanogruppe ist, in die Verbindung der Formel (I), worin $R_4$ eine Carboxamidogruppe ist, besteht.

7. Verfahren zur Herstellung von Pyridazinderivaten der Formel (I), worin $R_1$, $R_2$ und $R_4$ wie in Anspruch 1 definiert sind und Alk für eine 1,2–Propynylgruppe steht, dadurch gekennzeichnet, daß es darin besteht, daß das entsprechende, in Position 3 durch die Gruppe –NH–Alk, worin Alk eine 1,2– Propynylgruppe ist, substituierte Pyridazin einer Carbonatisierung unterzogen wird.

8. Verfahren nach einem der Ansprüche 1 bis 6, dadurch gekennzeichnet, daß man eine Verbindung der Formel 1 verwendet, in welcher $R_1$ die Gruppe 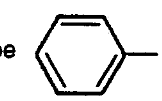 ist und $R_2$ Wasserstoff darstellt,

und daß man in Stufe 3) eine ω–halogenierte Verbindung der Formel X–Alk–$R_4$ verwendet, worin X ein Halogenatom ist, Alk für die Gruppe $-(CH_2)_3-$ steht und $R_4$ die Gruppe $-COOCH_3$ ist.

9. Verfahren nach einem der Ansprüche 1 bis 6, dadurch gekennzeichnet, daß man eine Verbindung der Formel 1 verwendet, in welcher $R_1$ die Gruppe 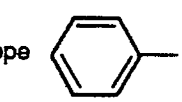 ist und $R_2$ für Wasserstoff steht, und daß man in Stufe 3) eine ω–halogenierte Verbindung der Formel X–Alk–$R_4$ verwendet, in welcher X ein Halogenatom ist, Alk für die Gruppe $-(CH_2)_3-$ steht und $R_4$ die Gruppe –COOH ist.

10. Verfahren zur Herstellung von Pyridazinderivaten, die auf das Zentralnervensystem aktiv sind, dadurch gekennzeichnet, daß es die folgenden Schritte umfaßt:

1) Umsetzung eines in Position 3 chlorierten Pyridazins der Formel 1:

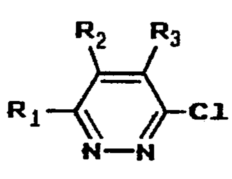

1

worin:

$R_1$ Wasserstoff, eine $C_1$–$C_4$–Alkylgruppe, eine Phenylgruppe, eine durch ein Halogen oder eine Nitrogruppe oder eine $C_1$–$C_4$– Alkylgruppe oder eine $C_1$–$C_4$–Alkoxygruppe oder eine Hydroxygruppe oder eine Trifluormethylgruppe monosubstituierte Phenylgruppe, eine durch ein Halogen disubstituierte Phenylgruppe, eine Naphthylgruppe, eine Cyclohexylgruppe, eine 2–Thienylgruppe, eine 3–Thienylgruppe oder eine 3–Indolylgruppe bezeichnet;

$R_2$ Wasserstoff, eine $C_1$–$C_4$–Alkylgruppe, eine Phenylgruppe darstellt;

$R_3$ für Wasserstoff, eine $C_1$–$C_4$–Alkylgruppe, eine Phenylgruppe steht;

mit einem Überschuß an Hydrazinhydrat $NH_2$–$NH_2$, um das Pyridazin der Formel 2:

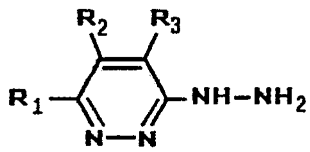

2

zu bilden, worin $R_1$, $R_2$ und $R_3$ wie oben definiert sind;

2) Überführung des Pyridazins der Formel 2 durch katalytische Hydrierung in das entsprechende 3–Aminopyridazin der Formel 3:

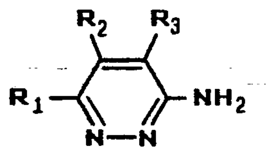

3

worin $R_1$, $R_2$ und $R_3$ wie oben definiert sind;

3) Umsetzung des 3–Aminopyridazins mit einer ω–halogenierten Verbindung der Formel X–Alk–$R_4$, worin X ein Wasserstoffatom ist und $R_4$ eine $C_1$–$C_4$–COO–Alkylgruppe oder die Cyanogruppe darstellt;

4) gegebenenfalls Überführung der so erhaltenen Verbindung durch Verseifung oder durch Einwirken von Ammoniak in einem aliphatischen Alkohol;

5) gegebenenfalls Überführung der erhaltenen Derivate in eines ihrer Additionssalze mit Säuren.

11. Verfahren nach Anspruch 10, dadurch gekennzeichnet, daß man in Stufe 1) das Pyridazin der Formel 2 verwendet,

worin $R_1$ die Gruppe 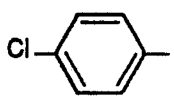 ist;

$R_2$ und $R_3$ Wasserstoff sind, und daß man in Stufe 3) die ω-halogenierte Verbindung der Formel X–Alk–$R_4$ verwendet, worin Alk die Gruppe $-(CH_2)_3-$ ist; X ein Halogen ist und $R_4$ die Gruppe $-COOC_2H_5$ darstellt, und daß man die so erhaltene Verbindung durch Verseifung in die entsprechende Säure überführt.

12. Verfahren nach Anspruch 10 , dadurch gekennzeichnet, daß man in der Stufe 1) das Pyridazin der Formel 2

verwendet, worin $R_1$ die Gruppe 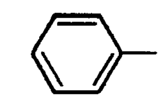 ist,

$R_2$ Wasserstoff ist und

$R_3$ für die Methylgruppe steht,

daß man in der Stufe 3) die ω-halogenierte Verbindung der Formel X–Alk–$R_4$ verwendet, worin Alk die Gruppe $-(CH_2)_3-$ ist, X ein Halogen ist und $R_4$ für die Gruppe $-COOC_2H_5$ steht, und daß man die so erhaltene Verbindung durch Verseifung in die entsprechende Säure überführt.

13. Verfahren zur Herstellung von Pyridazinderivaten, die auf das Zentralnervensystem aktiv sind, dadurch gekennzeichnet, daß es darin besteht, daß 3–Aminopyridazin der Formel 3 mit einem Halogenid X–Alk, worin Alk eine 1,2–Propynylgruppe ist, reagieren gelassen wird und das erhaltene Produkt einer Carbonatisierung unterzogen wird.

14. Verwendung der mittels des Verfahrens nach einem der Ansprüche 1 bis 13 erhaltenen Verbindungen zur Herstellung von Medikamenten.

15. Verwendung der mittels des Verfahrens nach einem der Ansprüche 1 bis 13 erhaltenen Verbindungen zur Herstellung von Medikamenten, die auf das Zentralnervensystem aktiv sind.

16. Verwendung nach einem der Ansprüche 14 oder 15 zur Herstellung von Medikamenten, die zur Verabreichung auf oralem Weg oder auf Injektionsweg konditioniert sind.

**Claims** for contracting States: BE, CH, DE, FR, GB, IT, LU, NL, SE

1. Pyridazine derivatives amino-substituted in the 3-position, characterized in that they correspond to the general formula:

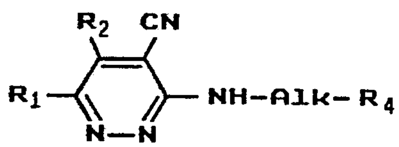

(I)

in which:

$R_1$ denotes hydrogen, a $C_1$–$C_4$ alkyl group, a phenyl group, a phenyl group monosubstituted by a halogen, a nitro group, or a $C_1$–$C_4$ alkyl group or a $C_1$–$C_4$ alkoxy group or a hydroxy group or a trifluoromethyl group, a phenyl group disubstituted by a halogen, a naphthyl group, a cyclohexyl group, a thien–2–yl group, a thien–3–yl group or an indol–3–yl group;

$R_2$ represents hydrogen, a $C_1$–$C_4$ alkyl group, a phenyl group;

Alk represents a group $(CH_2)_n$, in which n is an integer equal to 2, 3 or 4, or a 1, 2–propynyl group $-CH_2C{\equiv}C-$; and

$R_4$ represents: $-COOH$
$-COO-(C_1-C_4)$ alkyl
$-CONH_2$
$-C{\equiv}N$

and the addition salts of the said derivatives with acids.

2. Pyridazine derivatives according to claim 1, characterized in that:

$R_1$ is the group 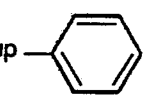

$R_2$ is hydrogen,
Alk is the group $-(CH_2)_3-$
$R_4$ is the group $-COOCH_3$.

3. Pyridazine derivative according to claim 1, characterized in that:

$R_1$ is the group 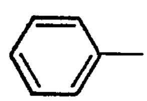

$R_2$ is hydrogen
Alk is the group $-(CH_2)_3-$ and
$R_4$ is the group $-COOH$.

4. Process for the preparation of the pyridazine derivatives of the formula (I) in which Alk is a group $(CH_2)_n$, according to claim 1, characterized in that it consists in:

1) reacting a pyridazine chlorinated in the 3-position, of the formula *1*:

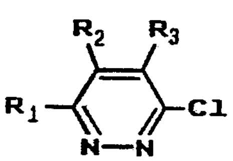

*1*

with an excess of hydrazine hydrate, $NH_2$–$NH_2$, to form the pyridazine of the formula *2*:

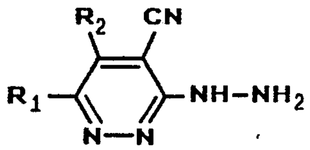

2

2) converting the pyridazine of the formula *2* to the corresponding 3–aminopyridazine of the formula *3* by catalytic hydrogenation;

3) reacting the said 3–aminopyridazine with an ω–halogeno compound of the formula X–Alk–$R_4$, in which X is a halogen atom and $R_4$ is a group COO–($C_1$–$C_4$)alkyl or the cyano group, to give the compound of the formula (I) in which $R_4$ is as defined above;

4) optionally converting the resulting compound of the formula (I) to a compound of the formula (I) in which $R_4$ is the COOH or $CONH_2$ group; and

5) optionally converting the derivatives obtained to addition salts with acids.

5. Process according to claim 4, characterized in that step 4) consists in saponifying the compound of the formula (I) in which $R_4$ is the group COO–($C_1$–$C_4$) alkyl to form the corresponding acid, namely the compound of the formula (I) in which $R_4$ is –COOH.

6. Process according to claim 5, characterized in that the saponification is carried out in an acid medium by known means.

7. Process according to claim 6, characterized in that the saponification is carried out by heating with a hydracid, in acetic acid, at a temperature of between 20 and 100°C.

8. Process according to claim 7, characterized in that the hydracid is hydrochloric or hydrobromic acid.

9. Process according to claim 4, characterized in that step 4) consists in converting the compound of the formula (I) in which $R_4$ is the group COO–($C_1$–$C_4$)alkyl or the cyano group to the compound of the formula (I) in which $R_4$ is a carboxamido group.

10. Process for the preparation of the pyridazine derivatives of the formula (I) in which $R_1$, $R_2$ and $R_4$ are as defined in claim 1, and Alk is a 1,2–propynyl group, characterized in that it consists in carbonating the corresponding pyridazine substituted in the 3–position by the group –NH–Alk in which Alk is a 1,2–propynyl group.

11. Pyridazine derivatives active on the central nervous system, characterized in that they are obtained by the process comprising the steps of:

1) reacting a pyridazine chlorinated in the 3–position, of the formula *1*:

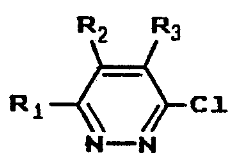

1

in which:

$R_1$ denotes hydrogen, a ($C_1$–$C_4$) alkyl group, a phenyl group, a phenyl group monosubstituted by a halogen or a nitro group or a $C_1$–$C_4$ alkyl group or a ($C_1$–$C_4$) alkoxy group or a hydroxyl group or a trifluoromethyl group, a phenyl group disubstituted by a halogen, a naphthyl group, a cyclohexyl group, a thien–2–yl group, a thien–3–yl group or an indol–3–yl group;

$R_2$ represents hydrogen, a ($C_1$–$C_4$), alkyl group, a phenyl group;

$R_3$ represents hydrogen, a ($C_1$–$C_4$) alkyl group, a phenyl group;

with an excess of hydrazine hydrate, $NH_2$–$NH_2$, to form the pyridazine of the formula *2*:

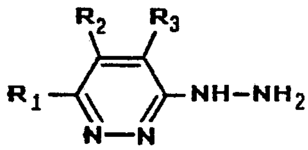

2

in which $R_1$, $R_2$ and $R_3$ are as above defined;

2) converting the pyridazine of the formula 2 to the corresponding 3–aminopyridazine of the formula *3* by catalytic hydrogenation:

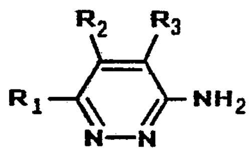

3

in which $R_1$, $R_2$ and $R_3$ are as defined above;

3) reacting the said 3–aminopyridazine with an ω–halogeno compound of the formula X–Alk–$R_4$, in which X is a halogen atom and $R_4$ is a group COO–($C_1$–$C_4$)alkyl or the cyano group;

4) optionally converting the resulting compound by saponification or by action of ammonia in an aliphatic alcohol.

5) optionally converting the derivatives obtained to addition salts with acids.

12. Derivative according to claim 11, characterized in that step 1) uses the pyridazine of formula *2* in which

$R_1$ is the radical

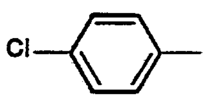

$R_2$ and $R_3$ denote hydrogen and in that in step 3) the ω–halogeno compound of formula X–Alk–$R_4$ in which Alk is the group -$(CH_2)_3$ is used; X is a halogen and $R_4$ is the group –$COOC_2H_5$ and in that the resulting compound is converted by saponification into the corresponding acid.

13. Derivative according to claim 11, characterized in that in step 1) the pyridazine of formula *2* in which

$R_1$ is the radical

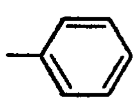

$R_2$ is hydrogen and

$R_3$ is the methyl group, is used and in that

in step 3) is used the ω–halogeno compound of formula X–Alk–$R_4$ in which Alk is the group –$(CH_2)_3$–; X is a halogen and $R_4$ is the group –$COOC_2H_5$ and in that the resulting compound is converted by saponification into the corresponding acid.

14. Pyridazine derivatives, characterized in that they are obtained by the process consisting in reacting the 3–amino pyridazine of formula 3 with an halide X–Alk in which Alk is a group 1,2–propynyl group and in subjecting the resulting product to a carbonating treatment.

15. Medecines, characterized in that they contain one of the pharmaceutically acceptable derivative of pyridazine according to any one of claims 1 to 3 and 11 to 14.

16. Medecines according to claim 15, characterized in that they are active on the central nervous system.

17. Medecines according to one of claims 15 or 16, characterized in that they are packed for oral administration or for injectable administration.

**Claims** for contracting State: AT

1. Process for obtaining pyridazine derivatives amino-substituted in the 3–position, characterized in that they correspond to the general formula:

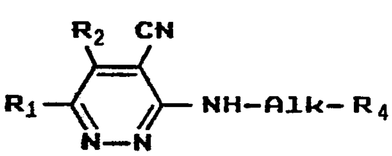

(I)

in which:

$R_1$ denotes hydrogen, a $C_1$–$C_4$ alkyl group, a phenyl group, a phenyl group monosubstituted by a halogen, or a nitro group, or a $C_1$–$C_4$ alkyl group or a $C_1$–$C_4$ alkoxy group or a hydroxy group or a trifluoromethyl

group, a phenyl group disubstituted by a halogen, a naphthyl group, a cyclohexyl group, a thien–2–yl group, a thien–3–yl group or an indol–3–yl group;

$R_2$ represents hydrogen, a $C_1$–$C_4$ alkyl group, a phenyl group;

Alk represents a group $(CH_2)_n$, in which n is an integer equal to 2, 3 or 4;

$R_4$ represents: –COOH

–COO–($C_1$–$C_4$)–alkyl

–$CONH_2$

–C≡N

and the addition salts of the said derivatives with acids, characterized in that it consists in:

1) reacting a pyridazine chlorinated in the 3–position, of the formula *1*:

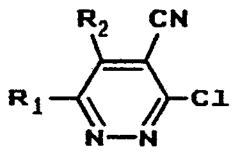

*1*

with an excess of hydrazine hydrate, $NH_2$–$NH_2$, to form the pyridazine of the formula *2*:

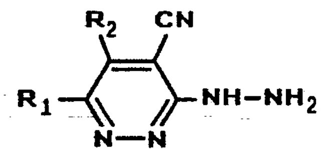

*2*

2) converting the pyridazine of the formula 2 to the corresponding 3–aminopyridazine of the formula *3* by catalytic hydrogenation;

3) reacting the said 3–aminopyridazine with an ω-halogeno compound of the formula X–Alk–$R_4$, in which X is a halogen atom and $R_4$ is a group COO–($C_1$–$C_4$)alkyl or the cyano group, to give the compound of the formula (I) in which $R_4$ is as defined above;

4) optionally converting the resulting compound of the formula (I) to a compound of the formula (I) in which $R_4$ is the COOH or $CONH_2$ group; and

5) optionally converting the derivatives obtained to addition salts with acids.

2. Process according to claim 1, characterized in that step 4) consists in saponifying the compound of the formula (I) in which $R_4$ is the group COO–($C_1C_4$)alkyl to form the corresponding acid, namely the compound of the formula (I) in which $R_4$ is –COOH.

3. Process according to claim 2, characterized in that the saponification is carried out in an acid medium by known means.

4. Process according to claim 3, characterized in that the saponification is carried out by heating with a hydracid, in acetic acid, at a temperature of between 20 and 100°C.

5. Process according to claim 4, characterized in that the hydracid is hydrochloric or hydrobromic acid.

6. Process according to claim 1, characterized in that step 4) consists in converting the compound of the formula (I) in which $R_4$ is the group COO–($C_1C_4$)–alkyl or the cyano group to the compound of the formula (I) in which $R_4$ is a carboxamido group.

7. Process for the preparation of the pyridazine derivatives of the formula (I) in which $R_1$, $R_2$ and $R_4$ are as defined in claim 1, and Alk is the group 1,2–propynyl group, characterized in that it consists in carbonating the corresponding pyridazine substituted in the 3–position by the group –NH–Alk in which Alk is a 1,2–propynyl group.

8. Process according to any one of claims 1 to 6, characterized in that a compound of formula 1 is used in which $R_1$ is the group

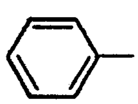

and $R_2$ is hydrogen, and in that in step 3) a ω–halogen compound is used of formula X–Alk–$R_4$, in which X is a halogen atom, Alk is the group –$(CH_2)_3$ and $R_4$ is the –$COOCH_3$ group.

9. Process according to any one of claims 1 to 6, characterized in that a compound of formula 1 is used in which $R_1$ is the group

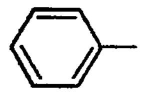

and $R_2$ is hydrogen, and in that in step 3), a –halogen compound is used, in which X is a halogen atom, Alk is the $-(CH_2)_3$ group and $R_4$ is $-COOH$.

10. Process for the preparation of pyridazine derivatives, active on the central nervous system, characterized in that it comprises the steps of:

1) reacting a pyridazine chlorinated in the 3-position, of the formula *1*:

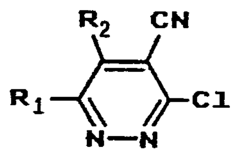

1

in which:

$R_1$ denotes hydrogen, a $C_1$–$C_4$ alkyl group, a phenyl group, a phenyl group monosubstituted by a halogen or a nitro group or a $C_1$–$C_4$ alkyl group or a $C_1$–$C_4$ alkoxy group or a hydroxy group or a trifluoromethyl group, a phenyl group disubstituted by a halogen, a naphthyl group, a cyclohexyl group, a thien–2–yl group, a thien–3–yl group or an indol–3–yl group;

$R_2$ represents hydrogen, a $C_1$–$C_4$ alkyl group, a phenyl group;

$R_3$ represents hydrogen, a $C_1$–$C_4$ alkyl group, a phenyl group;

with an excess of hydrazine hydrate, $NH_2$–$NH_2$, to form the pyridazine of the formula *2*:

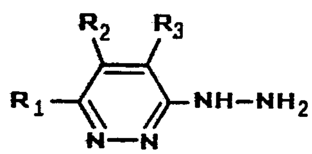

2

2) converting the pyridazine of the formula *2* to the corresponding 3–aminopyridazine of the formula *3* by catalytic hydrogenation:

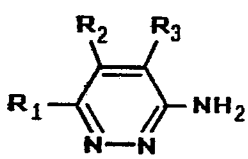

3

in which $R_1$, $R_2$ and $R_3$ are as defined above;

3) reacting the said 3–aminopyridazine with an ω–halogeno compound of the formula X–Alk–$R_4$, in which X is a halogen atom and $R_4$ is a COO–($C_1$–$C_4$)alkyl group or the cyano group;

4) optionally converting the resulting compound by saponification or by action of a ammonia in an aliphatic alcohol.

5) optionally converting the derivatives obtained to addition salts with acids.

11. Derivative according to claim 10, characterized in that step 1) uses the pyridazine of formula *2* in which

$R_1$ is the radical 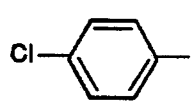

$R_2$ and $R_3$ denote hydrogen and in that step 3) uses the ω–halogeno compound of formula X–Alk–$R_4$ in which Alk is the group $-(CH_2)_3-$; X is a halogen and $R_4$ is the group $-COOC_2H_5$ and in that the resulting compound is converted by saponification into the corresponding acid.

12. Derivative according to claim 10, characterized in that in step 1) uses the pyridazine of formula *2* in which

$R_1$ is the radical 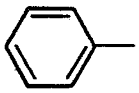

$R_2$ is hydrogen and
$R_3$ is the methyl group,

in that in step 3) is used the ω-halogeno compound of formula X–Alk–$R_4$ in which Alk is the group $-(CH_2)_3-$; X is a halogen and $R_4$ is the group $-COOC_2H_5$ and in that the resulting compound is converted by saponification into the corresponding acid.

13. Process for obtaining pyridazine derivatives active on the central nervous system, characterized in that it consists in reacting the 3–amino pyridazine of formula 3 with an halide X–Alk in which Alk is a group 1,2–propynyl group and in subjecting the resulting product to a carbonating treatment.

14. Uses of the compounds obtained by the process according to any one of claims 1 to 13 for producing medicines.

15. Use of medecines obtained by the process of any one of claims 1 to 13, for producing medecines which are active on the central nervous system.

16. Use according to one of claims 11 to 15, for producing medicines packed for oral administration or for injectable administration.